# EUROPEAN PATENT APPLICATION

(11) **EP 3 069 748 A1**
(43) Date of publication of application: **21.09.2016**
(21) Application number: 16160680.1
(22) Date of filing: 16.03.2016
(51) Int. Cl.: A61M 25/01, A61B 90/00

(54) **STEERABLE CATHETER**

(30) Priority: 19.03.2015 US 201562135444 P
(71) Applicant: Cook Medical Technologies LLC, Bloomington, IN 47404 (US)
(72) Inventor: QUEARRY, Benjaman B., Bloomington, IN 47403 (US)
(74) Representative: Garratt, Peter Douglas

(57) **Abstract**

A steerable catheter (10) that includes a first elongated member (12), a second elongated member (14), and a distal tip (46) coupled to distal portions of the first and second elongated members. The first elongated member has at least one lumen (16) and a male or female element (24, 26). The second elongated member has the other of the male or female element, wherein the male element engaged with the female element. Relative movement between the first elongated member and the second elongated member causes a curveable portion of the catheter proximal from the distal tip to bend without uncoupling of the first elongated member and the second elongated member. A sheath (20) may be provided to encircle the first and second elongated members and define the curveable portion between the distal end of the sheath and the distal tip of the catheter.

## Description

### CROSS-REFERENCE

The present application is a non-provisional application of, and claims priority under 35 U.S.C. § 120 to U.S. Provisional Application No. 62/135,444, "Steerable Cather," filed March 19, 2015.

### BACKGROUND

The field of the present invention relates to catheters for use in intraluminal passages. Catheters are commonly used for medical procedures within intraluminal passages. Commonly, navigating intraluminal passages may be difficult due to the narrowness of the passages, the curvature, and the branching configuration of the vasculature. To overcome these challenges, a thin, flexible wire guide is typically advanced first through the intraluminal passages and a catheter is advanced over the wire guide. However, in many circumstances, it is preferable to advance a catheter without a wire guide. A catheter which does not utilize a wire guide may have a smaller diameter than a catheter having a dedicated lumen for a wire guide. Additionally, most wire guides are made out of a metal or metal alloy, such as stainless steel, making them unavailable where magnetic resonance imaging is used in place of fluoroscopy.

### SUMMARY

Further areas of applicability will become apparent from the description provided herein. It should be understood that the description and specific examples are intended for the purpose of illustration only and are not intended to limit the scope of the present disclosure.

It is desirable that a catheter be capable of being advanced through tortuous intraluminal passages without being advanced over a wire guide or other catheter. It is further desirable that the catheter is steerable within intraluminal passages, and that the catheter is able to be used with magnetic resonance imaging as an alternative to fluoroscopy.

In one form of the present disclosure, a catheter is provided that includes a first elongated member, a second elongated member, and a distal tip coupled to the distal portions of the first elongated member and the second elongated member. The first elongated member has a lumen and one of a male and a female element. The second elongated member includes the other of the male and the female element wherein the male element engages with the female element to couple the first and second elongated members. Movement of the first elongated member relative to the second elongated member causes a curveable portion of the catheter proximal from the distal tip to bend without uncoupling the first elongated member and the second elongated member.

In another form of the present disclosure, a medical device is provided that includes a first elongated member and a second elongated member. The first elongated member has at least one lumen, a first distal end, and a male element. The second elongated member has a second distal end and a female element wherein the male element engages with the female element to slideably couple the first elongated member to the second elongated member. The first distal end is fixedly coupled to the second distal end so that advancement or retraction of one of the first elongated member or the second elongated member relative to the other of the first elongated member or the second elongated member causes a curveable portion of the device to bend without lateral separation of the first elongated member form the second elongated member.

### BRIEF DESCRIPTION OF THE DRAWINGS

The invention may be more fully understood by reading the following description in conjunction with the drawings, in which:
FIG. 1 is a cross-sectional view of a first embodiment of a steerable catheter including a first elongated member, a second elongated member, and a sheath;
FIG. 2 is a cross-sectional view of a second embodiment of the steerable catheter including a first elongated member and a second elongated member;
FIG. 3 is a cross-sectional view of a third embodiment of the steerable catheter including a first elongated member and a second elongated member;
FIG. 4 is a cross-sectional side view of a fourth embodiment of the steerable catheter including a first elongated member, a second elongated member, and a distal tip;
FIG. 5 is a cross-sectional side view of a fifth embodiment of the steerable catheter including a first elongated member, a second elongated member, and a distal tip;
FIG. 6 is a side view of an intraluminal passage including a sixth embodiment of the steerable catheter advancing through a branch of the intraluminal passage; and
FIGS. 7A-7D are side views of an intraluminal passage including a seventh embodiment of the steerable catheter advancing through a branch of the intraluminal passage.
The drawings described herein are for the purpose of illustration only and are not intended to limit the scope of the present disclosure in any way.

### DETAILED DESCRIPTION

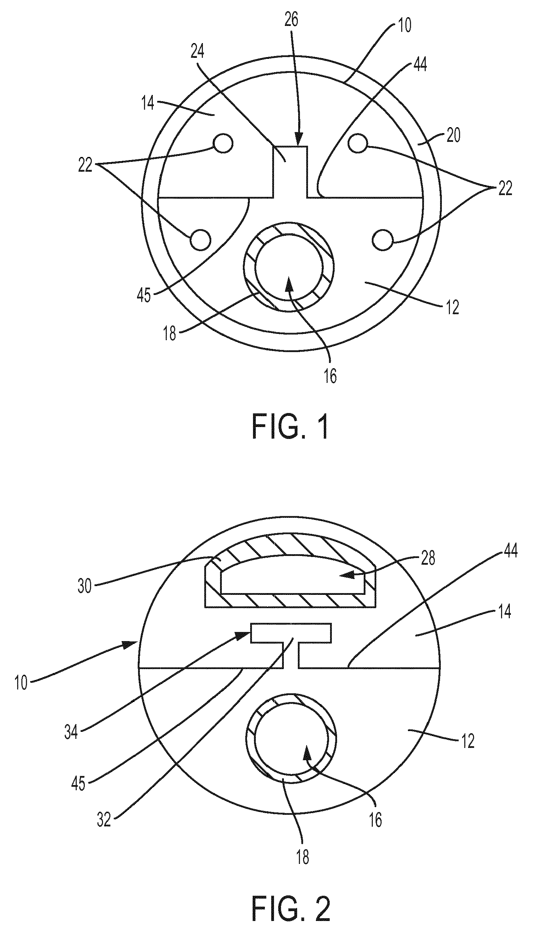

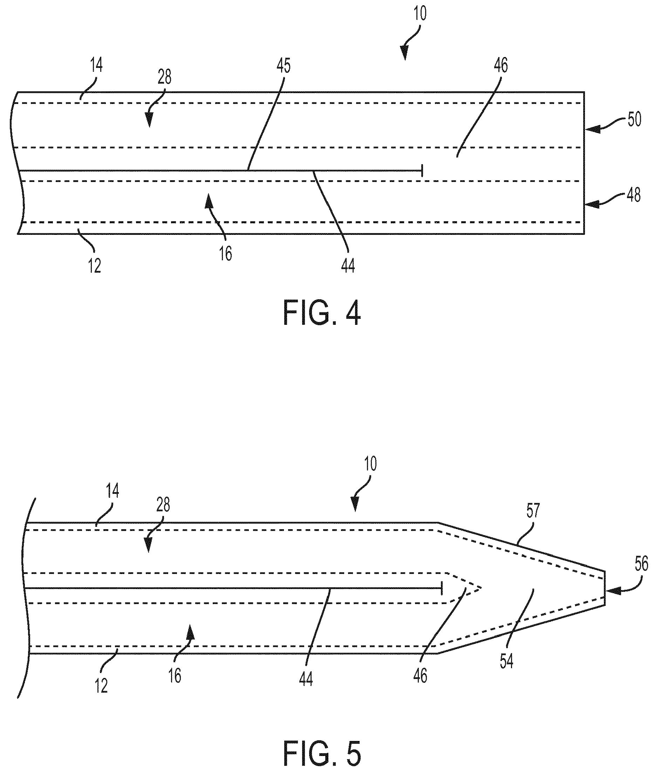

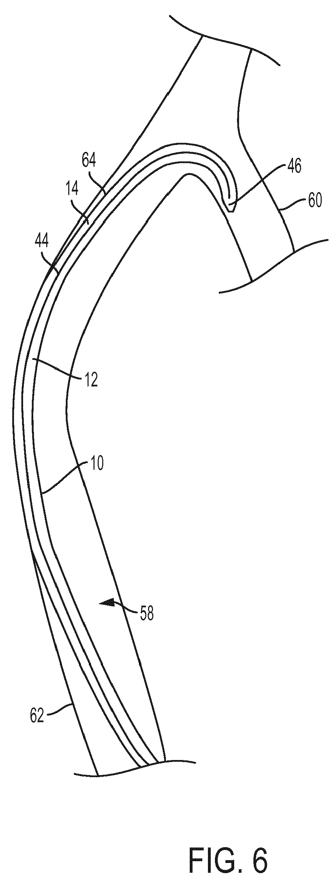

Referring now to the drawings, and particularly to FIG. 1, a steerable catheter 10 is shown including a first elongated member 12 having at least one lumen 16, and a second elongated member 14 coupled to the first elongated member 12. In this embodiment, the first elongated member 12 has one of either a male element 24 and a female element 26, and the second elongated member 14 has the other of the male element 24 and the female element 26. The distal portions of both of the first elongated member 12 and the second elongated member 14 are coupled to a distal tip (FIG. 4, 46) such that movement of the first elongated member 12 relative to the second elongated member 14 causes a curveable portion (FIG. 6, 64) to bend without uncoupling of the first elongated member 14 and the second elongated member. The relative movement between the first elongated member 12 and the second elongated member 14 may be advancement or retraction of either of the first and second elongated members 12, 14 while the other of the first and second elongated members 12, 14 is stationary, moving in the opposite direction, or moving in the same direction at a lower rate of motion.

The relative motion between the first and second elongated members 12, 14 may be initiated towards the proximal end of the catheter 10. In this embodiment, towards the proximal end of the catheter 10, the first and second elongated members 12, 14 are coupled only by the male element 24 and female element 26 interaction, and as a result, longitudinal relative movement between the first and second elongated members 12, 14 is possible. At the distal end of the catheter 10, the distal end of the first elongated member 12 is fixedly coupled to the distal end of the second elongated member 14. In the embodiment shown in FIG. 1, the distal ends of the first and second elongated members 12, 14 are coupled by a distal tip 46 (FIG. 4) which is coupled to the first and second elongated members 12, 14 to restrict longitudinal relative movement. As a result, any longitudinal relative movement between the first and second elongated members 12, 14 at the proximal end of the catheter 10 is translated into bending or curving of the first and second elongated members 12, 14 in the vicinity of the distal tip 46. As a non-limiting example of this function, advancing the first elongated member 12 relative to the second elongated member 14 will cause the first and second elongated members 12, 14 in the vicinity of the distal tip 46 to bend so that the second elongated member 14 is on the inside of the bend. If, instead, the first elongated member 12 is retracted, or the second elongated member 14 is advanced relative to the first elongated member 12, the opposite effect occurs, wherein the first and second elongated members 12, 14 in the vicinity of the distal tip 46 bends so that the first elongated member 12 will be on the inside of the bend. By this or similar movements, the direction of the distal tip 46 may be altered within the intraluminal passage (FIG. 6, 58).

In this embodiment, the first and second elongated members 12, 14 are coupled together by a male element 24 and a female element 26 to allow longitudinal relative movement between the first and second elongated members 12, 14. In this embodiment shown in FIG. 1, the male element 24 is a projection which is straight and rises from a first sliding surface 44 on the first elongated member 12. The projection 24 is received into a slot 26 which is formed on a second sliding surface 45 on the second elongated member 14. A projection 24 and slot 26 joint is relatively easy to manufacture and prevents the first and second elongated members 12, 14 from separating in a variety of directions. However, with the configuration shown in FIG. 1, the first and second elongated members 12, 14 may still separate in the direction of an applied curvature, unless an additional force is applied to the first and second elongated members 12, 14. At the distal end of the first and second elongated members 12, 14, this force is applied by the distal tip 46 which is coupled to both elongated members 12, 14. However, proximal from the distal tip 46, a sheath 20 may be used to surround both the first and second elongated members 12, 14 and prevent lateral separation even in the directions of curvature.

Additionally, the sheath 20 may be used to control the curvature of the catheter 10. The sheath 20, by fully encircling at least a portion of the first and second elongated members 12, 14, increases the overall rigidity of that portion of the first and second elongated members 12, 14, making that portion of the first and second elongated members 12, 14 more resistant to curvature. Therefore advancing or retracting the sheath 20 along the first and second elongated members 12, 14 defines a curveable portion 64 of the catheter 10 which is exposed between the distal end of the sheath 20 and the distal tip 46 of the catheter 10.

The embodiment shown in FIG. 1 includes a first elongated member 12 with a lumen 16 surrounded by a liner 18. The lumen 16 may be used for a variety of purposes, such as for deploying a smaller, follow-on device, delivering a drug to a specific area, aspirating a thrombus or blockage, or providing a pathway for a sensor to observe an area of an intraluminal passage 58. To minimize the diameter of the catheter 10, the lumen 16 may be placed within one of the first and second elongated member 12, 14 having the male element 24. In the embodiment shown in FIG. 1, the lumen 16 is arranged in a longitudinal direction, extending from a proximal end of the first elongated member 12 to the distal end of the first elongated member 12. Furthermore, the lumen 16 and the lumen liner 18 are fully enclosed within the first elongated member 12. Such a configuration may maximize the cross-sectional area available for the lumen 16, because the male element 24 projects into the female element 26 reducing the available cross-sectional area in which a lumen 16 may be placed.

Furthermore, the lumen liner 18 may be made from a different material than the first and second elongated members 12, 14, specifically one with a higher rigidity. It is possible that the inclusion of a lumen 16 within one of the first and second elongated members 12, 14 may change the overall curveability of the first and second elongated members 12, 14. When only one of the first and second elongated members 12, 14 includes a lumen 16, this difference in curveability between the first and second elongated members 12, 14 may cause bends within the curveable portion 64 to be uneven. Therefore, a liner 18 with a higher rigidity may be included surrounding the lumen 16 and balancing the overall curveability of the two elongated members 12, 14.

In the embodiment shown in FIG. 1, the first and second sliding surfaces 44, 45, of the first and second elongated members 12, 14 are shown as straight lines perpendicular to the direction of the projection 24 into the slot 26. Furthermore, the sliding surfaces 44, 45 may be configured to cross the maximum diameter of the catheter 10, ensuring that each elongated member 12, 14 has substantially equivalent cross-sectional areas, not including the cross-sectional area occupied by the slot 26 and projection 24. This arrangement may be desirable at least for simplicity of construction, but alternative arrangements may achieve different goals. The sliding surfaces 44, 45 may be curved so that one sliding surface 44, 45 has a convex curvature and the other has a concave curvature. In this way, the sliding surfaces 44, 45 may assist the male and female elements 24, 26 in preventing separation of the first and second elongated members 12, 14.

The first and second elongated members 12, 14 may be made of flexible materials such as polyvinyl chloride (PVC), or urethane. Liners 18 within the first and second elongated members 12, 14 may be made of materials such as nylon or other comparable materials. If the catheter 10 is designed to operate with magnetic resonance imaging (MRI), it may be desirable that the catheter 10 be made entirely from non-magnetic materials which might interfere with an MRI device. The associated sheath 20 may include a braid embedded within the walls of the sheath 20 to increase rigidity, however, such a braid may make the sheath 20 more difficult to advance over the catheter 10 within tortuous intraluminal passages 58. Furthermore, a braid made of a magnetic material such as stainless steel or nitinol may interfere with imaging methods such as MRI.

It may be desirable that the catheter 10 or other portions of the device be observable using ultrasound methodology while in operation. To accomplish this, echogenic materials 22 may be placed within the sheath 20 or within the first and second elongated members 12, 14. The echogenic materials 22 may be placed near the distal end of the sheath in order to assist the operator in adjusting the curveable portion 64 of the catheter 10. The echogenic materials 22 may be placed within the distal tip 46 to indicate to the operator the furthest advancement of the catheter 10 within the intraluminal passage 58. The echogenic materials 22 may also be placed along the length of either of the first and second elongated members 12, 14 to assist the operator in observing and adjusting the curvature of the curveable portion 64 of the catheter 10. The echogenic materials 22 may be glass, plastic beads, or tungsten powder, all of which have a resonance frequency which is readily distinct from the other materials of the device when exposed to ultrasonic frequencies.

Referring to FIG. 2, another possible embodiment of the catheter 10 is shown that includes a first elongated member 12 having a first lumen 16 and a male element 32, a second elongated member 14 having a second lumen 28 and a female element 34, wherein the male element 32 engages with the female element 34. The male element 32 shown in FIG. 2 is T-shaped male element 32, which fits within a T-shaped female element 34 to form a T-shaped joint. The T-shape arrangement of the male and female elements 32, 34 prevents separation of the first and second elongated members 12, 14 while still allowing for longitudinal relative movement between the first and second elongated members 12, 14. Therefore, the sheath 20 is not needed to prevent separation of the first and second elongated members 12, 14. The sheath 20 may still be desirable to better control the curvature of the curveable portion 64, however, even without a sheath 20, the catheter 10 shown in FIG. 2 will still have the bending curveable portion 64 when the first and second elongated members 12, 14 are moved relative to each other.

The embodiment of the device shown in FIG. 2 includes two lumens 16, 28, each having a lumen liner 18, 30. The first lumen 16 and the first liner 18 are disposed within the first elongated member 12. Because the male element 32 of the first elongated member 12 does not decrease the usable cross-sectional area of the first elongated member 12, it may be desirable to have the first lumen 16 be circular. A circular lumen may support a wider range of uses than a non-circular lumen, however, where each elongated member 12, 14 substantially forms a semicircle, the size of a circular lumen is limited to half of the radius of the catheter 10. As a result circular lumens within the first and second elongated members 12, 14 may be too small for some purposes, particularly where the cross-sectional area of one of the first and second elongated members 12, 14 is further limited by inclusion of the female element 34.

Therefore, the second elongated member 14 shown in FIG. 2 includes a second lumen 28 and a second liner 30 which is non-circular. Specifically in the embodiment shown, the second lumen 28 has a curved outer surface and a straight inner surface which are connected by sidewalls. While this arrangement of the second lumen 28 makes efficient use of the cross-sectional area of the second elongated member 14, other shapes may be preferable depending upon the use or ease of manufacture, such as an oval shaped lumen.

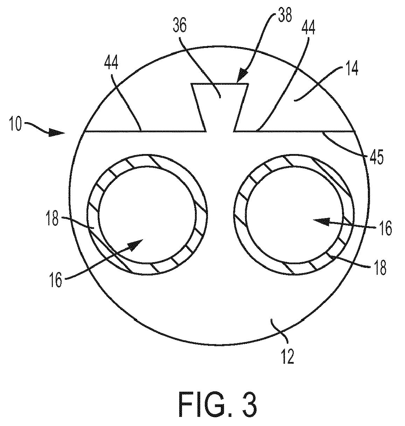

Referring to FIG. 3, another possible embodiment of the catheter 10 is shown including a first elongated member 12 having two lumens 16 surrounded by lumen liners 18, and a second elongated member 14 which has a smaller cross-sectional area than the first elongated member 12. In this embodiment, the first elongated member 12 includes a dovetail male element 36 which may engage with a dovetail female element 38 on the second elongated member 14. The dovetail male and female elements 36, 38 may form a dovetail joint which prevents separation of the first and second elongated members 12, 14, but allows longitudinal relative motion. Alternative shapes for the joint may function similarly to the dovetail male and female elements 36, 38 shown in FIG. 3, such as a bulbous tab.

In the dovetail joint shown in FIG. 3, the male element 36 has an increasing width with respect to the distance from the sliding surface 44 of the first elongated member 12. For example, where the male element 36 has an initial width where the male element 36 meets the sliding surface 44 of the first elongated member 12. The width of the male element 36 increases to an extended width as the male element 36 extends outward from the sliding surface 44. Similarly, in this embodiment, the shape of the female element 38 is closely matched to receive the male element 36. In order to ensure that the first and second elongated members 12, 14 remained joined under actuation, the female element 38 may have an initial gap width at the sliding surface 45 of the second elongated member 14 which is smaller than the extended width of the male element 36.

In some embodiments, the combined cross-sectional shape of the first elongated member 12 and the second elongated member 14 is substantially circular. In the embodiments shown in FIGS. 1 and 2, the sliding surfaces 44, 45 are arranged in a plane which passes through a diameter of the substantially circular cross-sectional shape. In this embodiment, the diameter of the substantially circular cross-sectional shape is the plane which extends across the widest point of substantially circular cross-sectional shape. As a result of this configuration, the cross-sectional shapes of the first and second elongated members 12, 14 are substantially semi-circular, excluding the male and female elements 32, 34.

However, the embodiment shown in FIG. 3 has sliding surfaces 44, 45 arranged in a plane which is offset from and parallel to the diameter of the substantially circular cross-sectional shape. As a consequence, the first elongated member 12 shown in FIG. 3 has a cross-sectional area which is substantially greater than a semi-circle, while the second elongated member 14 has a cross-sectional area which is less than a semi-circle.

The embodiment shown in FIG. 3 may be advantageous for several reasons. A first elongated member 12 may more efficiently position lumens 16 within the cross-sectional area, minimizing the size of the catheter 10 and enabling the catheter 10 to be utilized in narrow and tortuous intraluminal passages 58. The lumens 16 of the first elongated member may have a minimum size requirement to allow for adequate transmission of fluid through the lumen 16 or to allow a medical device to pass through the lumen 16. The first elongated member 12 have a greater cross-sectional area than the second elongated member 14 maximizes the size of the lumen 16 in the first elongated member 12, minimizing the size of the catheter 10.

In another example, in the embodiment shown in FIG. 3, the first elongated member 12 includes two lumens 16 in a side-by-side configuration, each having lumen liners 18. Because of the increased cross-sectional area in the first elongated member 12, which is also not reduced by the dovetail male element 36, both circular lumens 16 shown in FIG. 3 may be sized proportionally larger than comparable circular lumens 16 having first and second elongated members 12, 14 with substantially equivalent cross-sectional areas.

More generally, the percentage of the cross-sectional area of the first and second elongated members 12, 14 and the catheter 10 compared to the cross-sectional area of the lumens may be considered. For a substantially circular catheter 10 as shown in FIG. 3, where the first elongated member 12 occupies 75% of the total cross-sectional area of the catheter 10, two circular lumens 16 in a side by side configuration in the first elongated member 12 may occupy as much as about 60% of the cross-sectional area of the first elongated member 12 and about 45% of the total cross-sectional area of the catheter 10. Comparatively, a substantially circular catheter wherein the first and second elongated members 12, 14 occupy approximately equal portions of the cross-sectional area of the catheter 10, a similar side by side configuration of two circular lumens 16 in the first elongated member 12 may be expected to occupy a maximum of about 44% of the cross-sectional area of the first elongated member 12, and only about 22% of the total cross-sectional area of the catheter 10.

The percentage of the total catheter 10 cross-sectional area which is occupied by lumens 16 may be further increased above 60% by increasing the portion of the total cross-sectional area occupied by the first elongated member 12 and by including more lumens 16 within the first elongated member 12. By such methods, the efficiency of the use of catheter's 10 cross-sectional area may be maximized, and the catheter 10 may be designed to be as small as possible to accomplish its designed purpose within an intraluminal passage 58.

Additionally, the embodiment shown in FIG. 3, where the first elongated member 12 has a larger cross-sectional area than the second elongated member 14, may allow the curveable portion 64 to be more responsive in bending in a direction where the second elongated member 14 is on the inside of the bend. Because the second elongated member 14 has a smaller cross-sectional area, it may be more flexible when bending than the larger first elongated member 12. Therefore, retraction of the second elongated member 14 may cause a more responsive bend in the curveable portion 64 where the second elongated member 14 is on the inside of the bend. Comparatively, advancement of the second elongated member 14 may cause a comparatively less responsive bend in the curveable portion 64 due to the larger, less flexible first elongated member's 12 position on the inside of the bend.

In the embodiment shown in FIG. 4, the distal tip 46 is shown having both the first elongated member 12 and the second elongated member 14 coupled to the proximal portion of the distal tip 46. The sliding surfaces 44, 45 on each of the first and second elongated members 12, 14 terminate in or at the proximal portion of the distal tip 46. In the embodiment shown, the first and second lumens 16, 28 remain separated throughout the length of the distal tip 46 ending at the distal end of the distal tip through the first and second openings 48, 50. This configuration may be beneficial where each lumen 16, 28 is used for a separate purpose, and where both lumens 16, 28 may be used at once without interfering with each other. An example of such a configuration may be where thrombus retrieval device is deployed from the first lumen 16 and an embolic protection device is deployed from the second lumen 28.

In the embodiment shown in FIG. 5, an alternative embodiment of a distal tip 46 is shown which has an outer surface 57 which tapers distally. The tapered outer surface 57 terminates at a single distal opening 56. The distal tip 46 further contains a mixing chamber 54 defined by the outer surface 57. The mixing chamber 54 is in communication with both the first and second lumens 16, 28 as well as the distal opening 56. This configuration may be advantageous where only one lumen 16, 28 is going to be utilized at a time, where a device is deployed through one lumen 16, 28 while a fluid agent is deployed in the other lumen 16, 28, or where one of two different fluid agents are deployed in each lumen 16, 28 in order to mix within the mixing chamber 54 before proceeding into the intraluminal passage 58 through the distal opening 56.

In the embodiment shown in FIG. 6, an embodiment of the catheter 10 is shown within the intraluminal passage 58. As shown in FIG. 6, the catheter 10 is being advanced towards a desired branch 60 of the intraluminal passage which is at an angle from the main intraluminal passage 58. To bend the curveable portion 64 of the catheter 10 and point the distal tip 46 towards the desired branch 60, the first and second elongated members 12, 14 are moved relative to each other as described above. As a consequence of the relative movement of the first and second elongated members 12, 14, a bend is distributed along the entire curveable portion 64 of the catheter 10. Unlike the embodiment shown in FIG. 1, the embodiment shown in FIG. 6 does not include a sheath 20 and as a result, the curveable portion 64 includes the entire length of the first and second elongated members 12, 14. The bending of the first and second elongated members 12, 14 proximal to the distal tip 46 is constrained by the walls 62 of the intraluminal passage 58. A bend will be induced along the entire length of the first and second elongated members 12, 14, however, as the first and second elongated members 12, 14 are forced against the walls 62 of the intraluminal passage by this bending action, the majority of the bend will occur in the vicinity of the distal tip 46, allowing the distal tip 46 to advance through turns even at large angles to reach the desired branch 60.

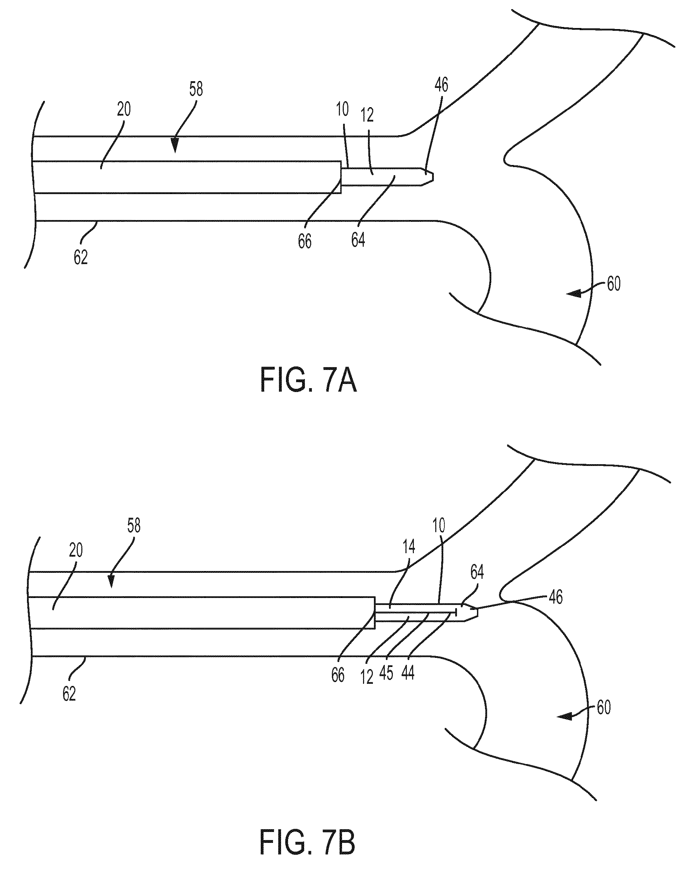

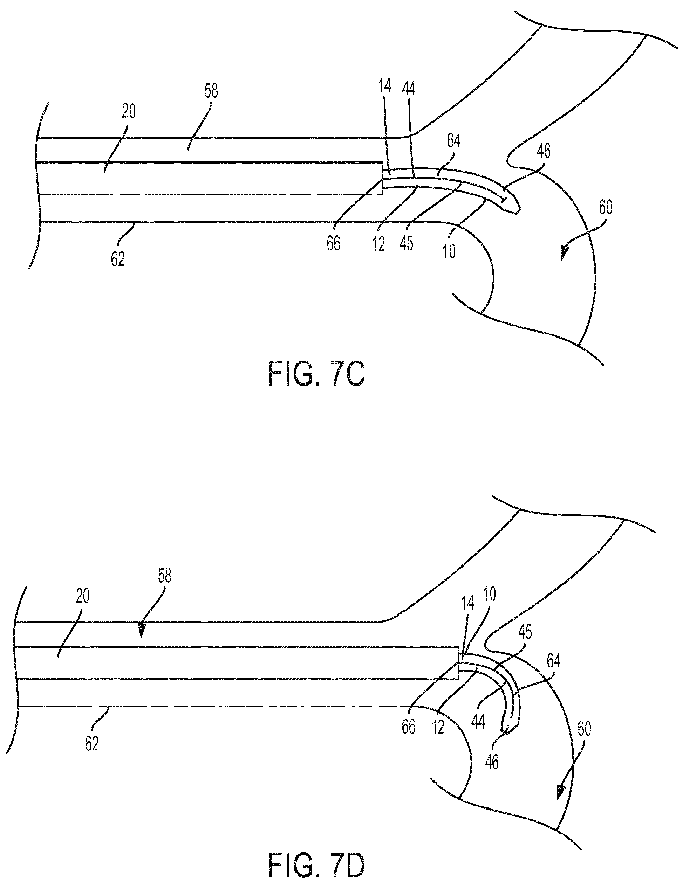

In the embodiment shown in FIGS. 7A-7D, the catheter 10 is within an intraluminal passage 58. In FIG. 7A, the catheter 10 is proximal from a branching intersection within the intraluminal passage 58. The desired branch 60 may have a more angularly severe curvature than other branches, therefore, without bending the device to choose the desired branch 60, it may be difficult to advance the catheter 10 into the desired branch 60. The embodiment of the catheter 10 shown in FIGS. 7A-7D includes a catheter 10 having a first elongated member 12 and a second elongated member 14, as well as a sheath 20 encircling the first and second elongated members 12, 14. FIG. 7A shows the catheter 10 wherein the first and second elongated members 12, 14 are not aligned to bend towards the desired branch 60.

FIG. 7B shows the catheter 10 being positioned so that the first and second elongated members 12, 14 are aligned with the desired branch 60. In order to bend towards the desired branch 60, the catheter 10 may need to be rotated so that the outer surface of one of the first or second elongated members 12, 14 is facing the desired branch. The outer surface of the first and second elongated members 12, 14 is defined as the surface which is opposite from the sliding surfaces 44, 45.

FIG. 7C shows the catheter 10 bending toward the desired branch 60. The bending of the catheter 10 occurs from relative movement between the first elongated member 12 and the second elongated member 14 actuated from the proximal end of one of the first and second elongated members 12, 14. The first and second elongated members 12, 14 having an outer surface which faces the desired branch 60 becomes the inside portion of the curveable portion 64, while the other elongated member 12, 14 becomes the outside portion of the curveable portion 64. While bending, the outer portion of the curveable portion 64 advances relative to the inside portion, and the inside portion retracts relative to the outside portion.

The radius of the catheter's 10 bend may be determined by the sheath 20. The curveable portion 64 is defined between the distal end 66 of the sheath 20 and the distal tip 46 of the catheter 10. While the catheter 10 is bending, the radius of the bend may be adjusted by advancing or retracting the sheath 20 so that the curveable portion 64 is larger or smaller. A larger curveable portion 64 will result in a bend having a larger radius, while a smaller curveable portion 64 will result in a bend having a smaller radius. As an alternative to moving the sheath 20, the catheter 10 may be advanced or retracted into the sheath 20 to control the radius of the bend. This may be desirable where an initial turn in the desired branch 60 is very sharp, but straightens out a short distance beyond. In such a situation advancing the catheter 10 while inducing a bend may allow the catheter 10 to initially have a bend with a small radius that increases as the catheter 10 advances into the desired branch 60. Once the distal tip 46 of the catheter 10 has advanced through the curvature of the desired branch, the actuation of the first and second elongated members 12, 14 may be released and the catheter 10 advanced further into the desired branch 60.

The curveable portion 64 may have a bend of up to 360 degrees of angular deflection. The radius of the bend may vary by the diameter of the catheter 10. For example, a bend of the curveable portion 64 may be expected to have a radius as small as 4 mm for a catheter 10 having a 3 mm diameter.

Accordingly, it is now apparent that there are many advantages of the invention provided herein. In addition to the advantages that have been described, it is also possible that there are still other advantages that are not currently recognized but which may become apparent at a later time.

While preferred embodiments of the invention have been described, it should be understood that the invention is not so limited, and modifications may be made without departing from the invention. The scope of the invention is defined by the appended claims, and all devices that come within the meaning of the claims, either literally or by equivalence, are intended to embrace them.

## Claims

1. A medical device, comprising:
a first elongated member having at least one lumen, a first distal end, and one of a male element and a female element; and
a second elongated member having a second distal end and the other of the male element and the female element, wherein the male element engages with the female element to couple the first elongated member to the second elongated member;
wherein the first distal end is directly or indirectly coupled to the second distal end such that advancement or retraction of one of the first elongated member or the second elongated member relative to the other of the first elongated member and the second elongated member causes a curveable portion of the device to bend.

2. The device of Claim 1, in the form of a catheter, comprising:
a distal tip coupled to the distal portions of the first elongated member and the second elongated member, wherein movement of the first elongated member relative to the second elongated member causes a curveable portion of the catheter proximal from the distal tip to bend while the first elongated member and the second elongated member remain coupled.

3. The device of Claim 2, further comprising a sheath encircling the first elongated member and the second elongated member.

4. The device of Claim 3, wherein a position of the sheath along the first and second elongated members defines the curveable portion of the catheter as the portion of the first and second elongated members which are located distally from a distal end of the sheath.

5. The device of any one of the preceding claims, wherein the male element and the female element engage in a straight projection and slot joint; in a T-shaped joint or in a dovetail joint.

6. The device of any one of the preceding claims, wherein the second elongated member comprises a second lumen.

7. The device of any one of the preceding claims, wherein a cross-sectional area of the second elongated member is smaller than a cross-sectional area of the first elongated member.

8. The device of Claim 2, wherein the catheter is made entirely of non-magnetic materials.

9. The device of Claim 2, wherein echogenic materials are embedded within the distal tip of the catheter.

10. The device of Claim 2, wherein the distal tip tapers distally to a distal opening.

11. The device of Claim 2, wherein at least two lumens in either of the first elongated member and the second elongated member converge within the distal tip.

12. The device of Claim 1 for use within an intraluminal passage,
wherein the first distal end is fixedly coupled to the second distal end such that advancement or retraction of one of the first elongated member or the second elongated member relative to the other of the first elongated member and the second elongated member causes a curveable portion of the device to bend without lateral separation of the first elongated member from the second elongated member.

13. The device of Claim 12, wherein the first elongated member comprises at least 75% of a total cross-sectional area of the catheter.

14. The device of Claim 12 or Claim 13, wherein the first elongated member comprises a plurality of lumens, preferably wherein the plurality of lumens comprise at least 50% of a cross-sectional area of the first elongated member.

15. The device of Claim 12, wherein the first elongated member further comprises a first sliding surface and the second elongated member further comprises a second sliding surface, wherein a portion of the first sliding surface contacts a portion of the second sliding surface, preferably wherein the male element extends from the first sliding surface and the female element is formed in the second sliding surface; preferably, wherein the male element comprises an initial width where the male element contacts the first sliding surface and an extended width where the male element is extended from the first sliding surface, wherein the initial width of the male element is smaller than the extended width of the male element; and preferably wherein the female element has an initial gap width at the second sliding surface and wherein the initial gap width of the female element is smaller than the extended width of the male element.
